# EUROPEAN PATENT APPLICATION

(11) **EP 4 282 954 A1**
(43) Date of publication of application: **29.11.2023**
(21) Application number: 21920449.2
(22) Date of filing: 23.03.2021
(51) Int. Cl.: C12N 5/10, C12N 15/85, C07K 16/24

(54) **CELL STRAIN FOR PRODUCING BIOSIMILAR DRUG OF USTEKINUMAB AND PRODUCTION METHOD THEREFOR**

(30) Priority: 25.01.2021 CN 202110099804
(71) Applicant: Qyuns Therapeutics Co., Ltd., Jiangsu 225300 (CN)
(72) Inventor: XU, Zhengxue, Taizhou, Jiangsu 225300 (CN); LI, Tao, Taizhou, Jiangsu 225300 (CN); CHEN, Yin, Taizhou, Jiangsu 225300 (CN); HUANG, Wenjun, Taizhou, Jiangsu 225300 (CN); WANG, Yi, Taizhou, Jiangsu 225300 (CN); QIAO, Huaiyao, Taizhou, Jiangsu 225300 (CN); FANG, Min, Taizhou, Jiangsu 225300 (CN); WU, Yiliang, Taizhou, Jiangsu 225300 (CN); ZHANG, Mengdan, Taizhou, Jiangsu 225300 (CN)
(74) Representative: Lavoix
(86) International application number: PCT/CN2021/082333
(87) International publication number: WO 2022/156060

(57) **Abstract**

Provided are a cell strain for producing a biosimilar drug of Ustekinumab and a production method therefor. Specifically, provided is a Chinese hamster ovary cell S cell strain, which is deposited in China Center for Type Culture Collection, with a deposit number of CCTCC NO: C2020232. The cell strain expresses a full human monoclonal antibody directed against the P40 subunit shared by human IL-12 and human IL-23. The fully human monoclonal antibody directed against the P40 subunit shared by human IL-12 and human IL-23 is a biosimilar drug of Ustekinumab, which not only exhibits high consistency with Ustekinumab in pre-clinical research, but also passes pharmacokinetic bioequivalence and safety similarity evaluation in clinical research. The biosimilar drug of Ustekinumab is the first one that has entered clinical trials in China, is the only one that has completed the I stage clinical trial, and is also one of the biosimilar drugs of Ustekinumab, which has the fastest progress in new drug application in the world.

## Description

### FIELD OF THE INVENTION

The application relates to the field of antibody medicine. Specifically, the present application relates to a Chinese hamster ovary cell S (CHO-S) cell line that can be used to produce a biosimilar drug of Ustekinumab (trade name Stelara^{®}) and the method for producing a biosimilar drug of Ustekinumab by using the CHO-S cell line.

### BACKGROUND OF THE INVENTION

Ustekinumab (trade name Stelara^{®}), developed by Johnson & Johnson, is a fully humanized monoclonal antibody targeting the p40 subunit shared by human IL-12 and human IL-23. IL-12 and IL-23 are heterodimeric cytokines expressed by activated antigen-presenting cells. IL-12 can activate NK cells to promote the differentiation of CD4⁺T cells into helper T cells (Th1), and IL-23 can activate the helper T cell 17 (Th17) pathway. Dysregulation of IL-12 and IL-23 is closely related to immune-related diseases. By blocking the binding of the p40 subunit shared by IL-12 and IL-23 to the IL-12Rβ1 to receptor protein on the surface of target cells, Ustekinumab inhibits the signal transduction and cytokine cascade mediated by IL-12 and IL-23, so as to exert curative effect in patients with autoimmune diseases such as psoriasis and inflammatory enteritis. Stelara^{®} was launched in Canada in 2008, and later in Europe, the United States, Japan and other places. The approved clinical indications include psoriasis, psoriatic arthritis, Crohn's disease, ulcerative colitis, etc.

Ustekinumab produced by Johnson & Johnson uses SP2/0 cells as host cells for expression. The antibody expressed by the SP2/0 cells has a relatively high proportion of sialic acid modification, and there is a certain risk of immunogenicity after entering the human body as drug. In theory, changing the host cell to express Ustekinumab may reduce the risk of immunogenicity. But in general, the same monoclonal antibody expressed by different types of host cells, even if the amino acid sequence is the same, there may be structural differences due to different glycosylation modes, etc. It is easy to cause differences in physical and chemical properties, biological activity, metabolic behavior and other aspects, resulting in low similarity between biosimilar drugs and original drugs, making it impossible to pass the similarity evaluation. This is a major difficulty in the development of biosimilar drugs, and it is also an important reason why there is little information about its biosimilar drugs, although the patent protection of Ustekinumab is about to expire.

### SUMMARY OF THE INVENTION

The purpose of the present application is to provide a cell line that can be used to produce a biosimilar drug highly similar to Ustekinumab in terms of key physical and chemical parameters, biological functions, etc., and a method for producing a biosimilar drug of Ustekinumab.

Specifically, the present application comprises:
1. A Chinese hamster ovary cell S (CHO-S) cell line, deposited with the China Center for Type Culture Collection (CCTCC), with a deposit number of CCTCC NO: C2020232; expressing a fully humanized monoclonal antibody against a p40 subunit shared by human IL-12 and human IL-23; comprising a heavy chain amino acid sequence of the fully humanized monoclonal antibody is shown as SEQ ID NO:1, and a light chain amino acid sequence of the fully humanized monoclonal antibody is shown as SEQ ID NO:3; comprising an expression plasmid that expresses the fully humanized monoclonal antibody against the p40 subunit shared by human IL-12 and human IL-23; the expression plasmid comprising a nucleotide sequence encoding the heavy chain amino acid sequence of the fully humanized monoclonal antibody shown as SEQ ID NO:2, and a nucleotide sequence encoding the light chain amino acid sequence of the fully humanized monoclonal antibody shown as SEQ ID NO:4.
2. The CHO-S cell line according to item 1, wherein the expressed fully humanized monoclonal antibody against the p40 subunit shared by human IL-12 and human IL-23 is substantially free of sialylated components.
3. The CHO-S cell line according to item 1, wherein the expressed fully humanized monoclonal antibody against the p40 subunit shared by human IL-12 and human IL-23 is a biosimilar drug of Ustekinumab.
4. A fully humanized monoclonal antibody against the p40 subunit shared by human IL-12 and human IL-23 expressed by the CHO-S cell line according to any one of items 1 to 3, wherein the fully humanized monoclonal antibody is a biosimilar drug of Ustekinumab.
5. The fully humanized monoclonal antibody against the p40 subunit shared by human IL-12 and human IL-23 according to item 5, wherein the fully humanized monoclonal antibody is substantially free of sialylated components.
6. A method for producing a biosimilar drug of Ustekinumab, comprising culturing the CHO-S cell line according to any one of items 1 to 3, thereby producing the fully humanized monoclonal antibody against the p40 subunit shared by human IL-12 and human IL-23 according to item 4 or 5.
7. An expression vector, comprising a DNA with a nucleotide sequence shown as SEQ ID NO:2 and a DNA with a nucleotide sequence shown as SEQ ID NO:4.
8. A host cell, comprising the expression vector according to item 7.

### TECHNICAL EFFECT

The above-mentioned Chinese hamster ovary cell S cell line was named as CHO-S-4, and was deposited in China Center for Type Culture Collection (CCTCC), with the preservation number CCTCC NO: C2020232. The biosimilar drug of Ustekinumab can be produced by culturing the Chinese hamster ovary cell S cell line to express the fully humanized monoclonal antibody against the p40 subunit shared by human L-12 and human IL-23. The biosimilar drug is highly similar to original Ustekinumab in terms of key physical and chemical parameters and biological functions, etc. The biosimilar drug of Ustekinumab (named as QX001S) expressed by the CHO-S cell line not only showed a high degree of consistency with Ustekinumab in preclinical studies, but also passed the evaluation of pharmacokinetic bioequivalence and safety similarity in clinical studies, has become the first biosimilar drugs of Ustekinumab to enter the clinical trial and the only one to complete the Phase I clinical trial in China so far. As far as we know, it is also one of the biosimilar drugs of Ustekinumab with the fastest progress in new drug application internationally.

### BRIEF DESCRIPTION OF THE DRAWINGS

Figure 1 shows the amino acid sequence of the heavy chain and the amino acid sequence of the light chain of Ustekinumab.
Figure 2 shows the main pharmacokinetic parameters and plasma concentration-time variation of QX001S and Ustekinumab administered by single subcutaneous injection of 1mg/kg group in Cynomolgus monkeys.
Figure 3 shows the main pharmacokinetic parameters and plasma concentration-time variation of QX001S and Ustekinumab administered by single subcutaneous injection in human body, that is, 90% confidence interval (CI) for the geometric mean ratio of Cₘₐₓ, AUC₀₋ₜ and AUC_{0-inf}.
Figure 4 is the heavy chain gene sequence of QX001S with codon optimization.
Figure 5 is the light chain gene sequence of QX001S with codon optimization.

### DETAIL DESCRIPTION OF THE INVENTION

### Definitions

The term "cell line" as used herein refers to cultured cells with special properties or markers obtained from primary cultures or cell lines by selection or clone formation. The special properties or markers of the cell line must persist throughout the culture period. The genetic material of the cell line is unchanged.

The term "DNA" as used herein refers to a macromolecular polymer composed of deoxyribonucleotides. Deoxyribonucleotides are composed of bases, deoxyribose and phosphoric acid. There are four kinds of bases: adenine (A), guanine (G), thymine (T) and cytosine (C). In the molecular structure, two polydeoxynucleotide chains coil around a common central axis to form a double helix structure. The deoxyribose-phosphate chains are outside the helix, with the bases facing inside. The two deoxyribonucleotide chains complement each other in reverse, connected by base pairing formed by hydrogen bonds between bases, forming a fairly stable combination. DNA carries the genetic information necessary for the synthesis of RNA and protein, and is an essential biological macromolecule for the development and normal operation of organisms.

The term "expression vector" as used herein refers to a vector that adds expression elements (such as promoter, ribosome binding site, terminator, etc.) on the basis of the basic skeleton of the cloning vector to enable the expression of the target gene.

The term "monoclonal antibody" as used herein refers to a highly homogeneous antibody produced by a single B cell clone that only targets a specific antigenic epitope. It is usually prepared by hybridoma technology. Hybridoma antibody technology is based on cell fusion technology, which fuses sensitized B cells with the ability to secrete specific antibodies and myeloma cells with unlimited reproductive capacity into B-cell hybridoma. By culturing a single hybridoma cell with this characteristic into a cell population, a specific antibody, i.e. monoclonal antibody, targeting a specific antigen epitope can be prepared.

The term "humanized antibody" as used herein, also known as complementarity-determining region-grafted antibody, refers to the variable region part of the antibody or all of the antibody being encoded by the human antibody genes.

The term "humanized monoclonal antibody" used herein refers to an antibody that can maintain their specificity and majority of affinity while almost eliminating immunogenicity and toxic side effects through affinity remodeling by grafting the antibody fragment (complementarity determining region, CDR) that directly contacts the countless existing mouse antibodies that have been analyzed in detail with the antigen onto the human antibody framework.

The term "fully humanized antibody" as used herein refers to the transfer of human antibody genes through transgenic or transchromosomal technology to transfer all human antibody-encoding genes into genetically engineered animals lacking antibody genes, so that the animals express human antibodies, to achieve the purpose of full humanization of antibodies. A variety of methods have been established to produce fully humanized antibodies, mainly including phage display technology, transgenic mouse technology, ribosome display technology and RNA-peptide technology.

The present application relates to a Chinese hamster ovary cell S (CHO-S) cell line named as CHO-S-4, which is deposited in the China Center for Type Culture Collection (CCTCC), the deposit number is CCTCC NO: C2020232, and the deposit date is December 03, 2020.

In a specific embodiment, the Chinese hamster ovary cell S (CHO-S) cell line expresses a fully humanized monoclonal antibody against the p40 subunit of human IL-12/IL-23, and the amino acid sequence of the heavy chain of the fully humanized monoclonal antibody is the amino acid sequence of the heavy chain of Ustekinumab, and the amino acid sequence of the light chain of the fully humanized monoclonal antibody is the amino acid sequence of the light chain of Ustekinumab.

In a specific embodiment, the Chinese hamster ovary cell S (CHO-S) cell line expresses a fully humanized monoclonal antibody against the p40 subunit of human IL-12/IL-23, and the amino acid sequence of the heavy chain of the fully humanized monoclonal antibody is shown as SEQ ID NO:1, and the amino acid sequence of the light chain of the fully humanized monoclonal antibody is shown as SEQ ID NO:3.
SEQ ID NO: 1 
EVQLVQSGAEVKKPGESLKISCKGSGYSFTTYWLGWVRQMPGKGLDWIGIM
SPVDSDIRYSPSFQGQVTMSVDKSITTAYLQWNSLKASDTAMYYCARRRPGQ
GYFDFWGQGTLVTVSSSSTKGPSVFPLAPSSKSTSGGTAALGCLVKDYFPEPV
TVSWNSGALTSGVHTFPAVLQSSGLYSLSSVVTVPSSSLGTQTYICNVNHKPS
NTKVDKRVEPKSCDKTHTCPPCPAPELLGGPSVFLFPPKPKDTLMISRTPEVTC
VVVDVSHEDPEVKFNWYVDGVEVHNAKTKPREEQYNSTYRVVSVLTVLHQ
DWLNGKEYKCKVSNKALPAPIEKTISKAKGQPREPQVYTLPPSRDELTKNQV
SLTCLVKGFYPSDIAVEWESNGQPENNYKTTPPVLDSDGSFFLYSKLTVDKSR
WQQGNVFSCSVMHEALHNHYTQKSLSLSPGK
SEQ ID NO:3

DIQMTQSPSSLSASVGDRVTITCRASQGISSWLAWYQQKPEKAPKSLIYAASSL
QSGVPSRFSGSGSGTDFTLTISSLQPEDFATYYCQQYNIYPYTFGQGTKLEIKRT
VAAPSVFIFPPSDEQLKSGTASVVCLLNNFYPREAKVQWKVDNALQSGNSQES
VTEQDSKDSTYSLSSTLTLSKADYEKHKVYACEVTHQGLSSPVTKSFNRGEC

In a specific embodiment, the Chinese hamster ovary cell S (CHO-S) cell line comprises an expression plasmid expressing the fully humanized monoclonal antibody against the p40 subunit shared by human IL-12 and human IL-23. The expression plasmid comprises the nucleotide sequence shown as SEQ ID NO:2 encoding the amino acid sequence of the heavy chain of the fully humanized monoclonal antibody, and the nucleotide sequence shown as SEQ ID NO:4 encoding the amino acid sequence of the light chain of the fully humanized monoclonal antibody.
SEQ ID NO:2 
GAAGTCCAGCTGGTGCAGTCAGGGGCCGAGGTGAAGAAACCAGGTGAAA
GTCTGAAGATCAGCTGTAAAGGCTCTGGATACAGTTTCACCACATATTGGCT
GGGATGGGTGCGGCAGATGCCCGGGAAGGGTCTGGATTGGATCGGTATTAT
GTCCCCCGTGGACAGCGACATCAGATACAGTCCTTCATTTCAGGGCCAGGT
CACTATGTCTGTGGACAAGAGTATTACTACCGCTTATCTGCAGTGGAACTCA
CTGAAAGCCTCCGATACCGCTATGTACTATTGCGCAAGGCGGAGACCCGGC
CAGGGATACTTCGACTTTTGGGGGCAGGGCACCCTGGTCACAGTGTCCAG
CTCTAGTACTAAGGGGCCTTCCGTGTTTCCACTGGCTCCCTCATCCAAATCC
ACTAGCGGAGGAACCGCAGCTCTGGGATGTCTGGTGAAGGACTATTTCCCA
GAGCCCGTCACAGTGTCATGGAACTCCGGCGCACTGACCAGCGGAGTCCA
TACATTTCCTGCCGTGCTGCAGAGCTCTGGGCTGTACTCTCTGAGTTCAGTG
GTCACAGTGCCATCCAGCTCTCTGGGTACTCAGACCTATATCTGCAACGTG
AATCACAAGCCTTCTAATACTAAGGTCGATAAGAGGGTGGAACCAAAGAGT
TGTGATAAAACACATACTTGCCCCCCTTGTCCTGCACCAGAGCTGCTGGGG
GGTCCAAGCGTGTTCCTGTTTCCACCCAAGCCCAAAGATACCCTGATGATT
 
TCCCGAACACCAGAAGTCACTTGCGTGGTCGTGGACGTGAGCCACGAGGA
CCCCGAAGTCAAGTTCAACTGGTACGTGGACGGCGTCGAGGTGCATAATGC
TAAGACCAAACCTCGCGAGGAACAGTACAATAGCACATATCGAGTCGTGTC
TGTCCTGACTGTGCTGCACCAGGATTGGCTGAACGGAAAGGAGTATAAGTG
CAAAGTGTCTAATAAGGCTCTGCCCGCACCTATCGAGAAAACCATTTCCAA
GGCCAAAGGCCAGCCTCGTGAACCACAGGTGTACACACTGCCTCCATCAA
GGGATGAGCTGACTAAGAACCAAGTCAGCCTGACCTGTCTGGTGAAAGGC
TTCTATCCCTCTGACATCGCTGTGGAGTGGGAAAGTAATGGACAGCCTGAA
AACAATTACAAGACAACTCCCCCTGTGCTGGACAGCGATGGCTCTTTCTTT
CTGTATTCCAAGCTGACCGTGGACAAAAGCCGGTGGCAGCAGGGAAACGT
CTTTTCTTGTAGTGTGATGCATGAGGCCCTGCACAATCATTACACACAGAAG
TCACTGTCCCTGAGCCCAGGAAAA
SEQ ID NO:4

GACATCCAGATGACCCAGAGTCCATCCAGCCTGTCTGCTAGTGTGGGA
GATAGGGTCACTATCACCTGTCGGGCCTCCCAGGGGATTTCTAGTTGG
CTGGCTTGGTATCAGCAGAAGCCAGAGAAAGCACCCAAGTCCCTGATT
TATGCCGCTTCATCCCTGCAGAGTGGAGTGCCCTCACGATTCTCAGGC
TCCGGAAGCGGGACTGACTTTACACTGACTATCAGCTCTCTGCAGCCT
GAAGATTTCGCTACCTACTATTGCCAGCAGTACAACATTTACCCATATA
CTTTTGGTCAGGGCACCAAACTGGAGATCAAGAGAACAGTGGCAGCC
CCCAGCGTCTTCATTTTTCCCCCTTCTGACGAACAGCTGAAATCCGGC
ACCGCAAGCGTGGTCTGTCTGCTGAACAATTTCTACCCTCGCGAGGCC
AAAGTGCAGTGGAAGGTCGATAACGCTCTGCAGTCTGGCAATAGTCA
GGAGTCAGTGACAGAACAGGACTCCAAAGATAGCACTTATTCTCTGAG
TTCAACCCTGACACTGAGCAAGGCAGACTACGAGAAGCACAAAGTGT
ATGCCTGCGAAGTCACCCATCAGGGTCTGTCCAGCCCCGTGACAAAG
TCTTTTAATAGGGGCGAATGT

In a specific embodiment, the Chinese hamster ovary cell S (CHO-S) cell line expresses a fully humanized monoclonal antibody against the p40 subunit shared by human IL-12 and human IL-23, and the fully humanized monoclonal antibody is substantially free of sialylated components.

The present application also relates to a fully humanized monoclonal antibody against the p40 subunit shared by human IL-12 and human IL-23 expressed by the above-mentioned CHO-S cell line, which is a biosimilar drug of Ustekinumab (named as QX001S), and is highly similar to Ustekinumab as the original drug.

Specifically, the primary, secondary and advanced structures of QX001S are similar to those of Ustekinumab; for both QX001S and Ustekinumab, the glycosylation modification sites are similar; the analysis data of charge heterogeneity, such as the sugar-free isoelectric point, acidic peak, and alkaline peak, are similar; the data of polymer and degradation product analysis are similar; the thermal stability is similar; the analysis data of in vitro biological activity, such as antigen IL-12 binding activity, antigen IL-12 affinity, antigen IL-23 binding activity, antigen IL-23 affinity , IL-12 receptor antagonistic activity, IL-23 receptor antagonistic activity, NK-92 MI cell activity, mouse spleen cell activity, Fc-related receptor affinity, species specificity analysis, antigen recognition epitope consistency analysis and so on, are similar.

Pharmacodynamic studies in the mouse ear epidermal hyperplasia model induced by IL-23 showed that the QX001S group and the Ustekinumab group showed highly similar consistency in their pharmacological effects. In vivo pharmacokinetic studies of Cynomolgus monkeys showed that both Ustekinumab and QX001S had similar pharmacokinetic parameters and plasma concentration-time variation in Cynomolgus monkeys *in vivo.* In vivo pharmacokinetic studies of humans show that the pharmacokinetics of QX001S injection and Ustekinumab injection are bioequivalent and have similar safety profiles.

In addition, the present application also relates to a method for producting a biosimilar drug of Ustekinumab, comprising culturing the CHO-S cell line, thereby producing the fully humanized monoclonal antibody against the p40 subunit shared by human IL-12 and human IL-23.

### EXAMPLE

Hereinafter, the present application will be described more specifically by examples. It should be understood that the present application is not limited to these examples.

### Example 1: vector construction

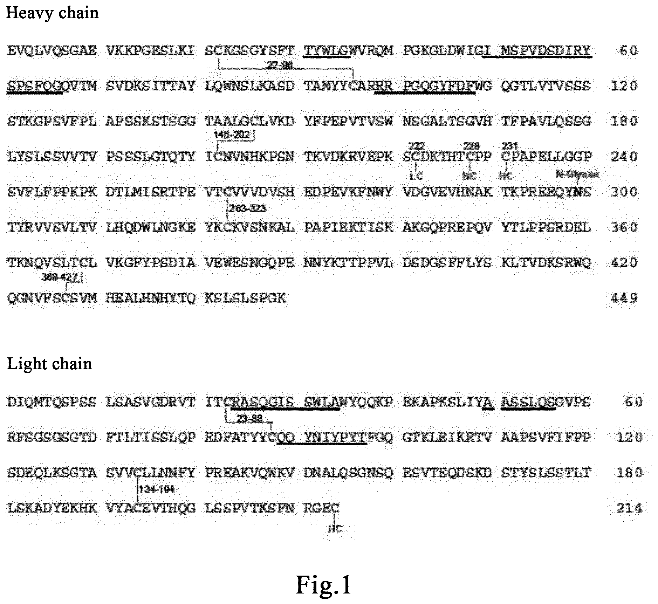

The amino acid sequence of the heavy chain (SEQ ID NO:1) and the amino acid sequence of the light chain (SEQ ID NO:3) of Ustekinumab were determined through public information query and peptide analysis. (Figure 1)

The amino acid sequence of the heavy chain and the amino acid sequence of the light chain of QX001S, which is a biosimilar drug of Ustekinumab, are completely identical to those of Ustekinumab.

The gene sequences of the heavy chain and light chain of QX001S was designed using the amino acid sequence of the heavy chain and light chain of Ustekinumab as templates, and the expression plasmid was constructed.

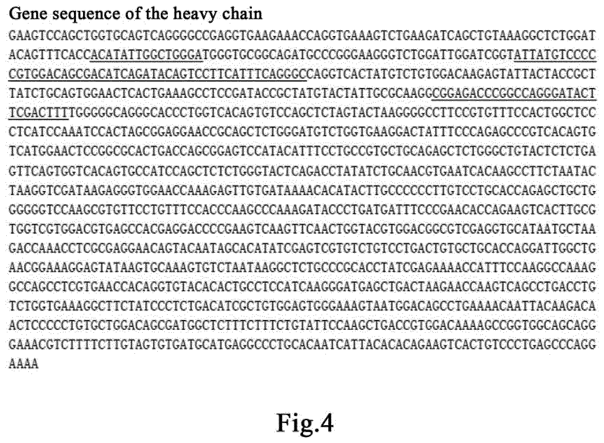

The codon-optimized gene sequence of the heavy chain of QX001S is SEQ ID NO:2 (as shown in Figure 4).

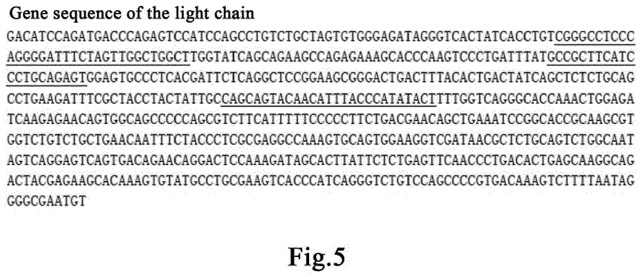

The codon-optimized gene sequence of the light chain of QX001S is SEQ ID NO:4 (as shown in Figure 5).

The expression plasmid was constructed by insertion of the codon-optimized heavy chain gene and light chain gene of QX001S respectively into their respective blank expression vectors through well-known molecular biology methods, and connected them with the upstream and downstream expression regulatory elements; then the heavy chain and its expression regulatory elements were enzymatically cleaved and connected to the light chain expression vector to form a heavy chain and light chain linked plasmid, which is the final expression plasmid used for transfection and construction of engineered cells. The final expression plasmid also contains the screening marker glutamine synthetase (GS) gene, which is widely used in the construction of engineered cell lines using CHO cells, and can be used for subsequent screening of stably transfected cells.

Transient transfection and expression was performed for the expression plasmid obtained above, and then, protein sequence identification and activity analysis were performed on the expressed QX001S to confirm that the gene sequence of the constructed QX001S expression vector and the expressed protein were correct.

### Example 2: Screening of cell lines

The gene sequence of the constructed QX001S expression vector and the expressed protein were confirmed correct, and CHO-S was used as the host cell to construct the stable expression cell line of QX001S. The CHO-S host cells were purchased from ThermoFisher (formerly Invitrogen, Cat. No. A13696-01), recovered and cultured the CHO-S^{™} cells. After electroporation of the linearized expression vector into the host cell, it was randomly integrated into the host cell genome through homologous arms on the vector.

Using glutamine synthetase as a screening marker, a QX001S CHO-S cell line with stable, high-yield expression and physicochemical properties consistent with Ustekinumab was obtained by pressurized screening with methioninesulfoximine (MSX).
1. Through step-by-step amplification, ELISA detection, etc., for the cell clones cultured in 40 of 96-well plates, a high-yield cell pool (mixed cell clones) was obtained by preliminary screening, and 14 high-yield cell pools were selected as the first round of subcloning candidate pools.
2. The obtained 14 cell pools were not monoclonal, and these cells needed to be monoclonalized to obtain a single stable cell line. 14 high-yield cell pools were subcloned by limiting dilution method, and high-expression single clones were screened out through step-by-step amplification and ELISA detection. A total of 694 cell clones were amplified, and finally 33 clones with the highest OD value were selected and expanded to suspension culture in T25 (8ml of CD CHO medium containing 100µmol/L MSX). Cell supernatant was harvested after 8 days of cell culture in T25, IgG content was detected by Fortebio, and QX001S was purified with Protein A for quality analysis.
3. Considering that the quality attributes of the expressed QX001S should be consistent with those of Ustekinumab, for the above 33 monoclonals, the clones with simple alkaline components and low content of the secreted QX001S after carboxypeptidase treatment were selected. Clone 4, Clone 9, Clone 25 and Clone 27 were selected. Furthermore, peptide map analysis was performed to compare the QX001S expressed by the three clones 4, 25, and 27 with Ulinumab. The peptide maps of the four samples were basically the same, and there was no significant increase or decrease in peptide segments, indicating that the amino acid sequences of QX001S secreted by the above 3 clones were correct.
4. In order to obtain a uniform, stable and high-yielding QX001S producing cell line, a second round of subcloning screening was performed on the above four candidate clones. The cell state of clone 25 did not meet the requirements during the second round of subcloning screening; the yield of clones obtained after the second round of subcloning of clone 27 was low; clone 9 did not meet the requirements in the preliminary stability test; the above three clones were eliminated.

That is, among the 33 clones that entered the quality analysis, only one clone of clone 4 met the requirements as QX001S producing cell line.

The CHO-S cell line of clone 4 was named as Chinese hamster ovary cell CHO-S-4, which was preserved in the China Center for Type Culture Collection (CCTCC), with the deposit number CCTCC NO: C2020232, and the deposit date was December 03, 2020. The fully humanized monoclonal antibody against the p40 subunit of human IL-12/IL-23 expressed by the CHO-S cell line of clone 4 was named as QX001S.

### Example 3: pharmaceutical similarity research

The pharmaceutical similarity study between QX001S expressed by the CHO-S cell line of the above clone 4 and Ustekinumab was performed. The similarities and differences in the primary structure, secondary and advanced structures, post translational modifications, charge heterogeneity, polymers and degradation products, in vitro biological activity, and accelerated stability between QX001S and Ustekinumab were comprehensively compared, the results indicated that the two were highly similar in pharmaceutical research. QX001S expressed by the CHO-S cell line is substantially consistent with Ustekinumab in 71 of the 78 indicators listed in Table 1 below.

The indicators that have differences between the two are mainly in post-translational modification (there are differences in 5 indicators), especially as determined, the proportion of sialylated components of Ustekinumab is between 11.87% and 25.56%, while QX001S expressed by the CHO-S cell line is substantially free of sialylated components, suggesting that QX001S may have lower immunogenicity than Ustekinumab.

**Table 1: summary table of indicators of pharmaceutical similarity research**

| **classification** | **quality parameter** | | | **similarity assessment** |
|---|---|---|---|---|
| Primary structure | Ratio of amino acid coverage | | | Preset range |
| | Profile of ratio of amino acid coverage | | | Qualitative Comparison |
| | N-terminal sequence | | | Preset range |
| | Secondary profile of N-terminal sequence | | | Qualitative comparison |
| | C-terminal sequence | | | Preset range |
| | Secondary profile of C-terminal sequence | | | Qualitative comparison |
| | Profile of complete protein MW (desaccharification) | | | Qualitative comparison |
| | Complete protein molecular weight (desaccharification) | | | Preset range |
| | Profile of reduction (desaccharification) molecular weight | | | Qualitative comparison |
| | Reduction (desaccharification) molecular weight: LC | | | Preset range |
| | Reduction (desaccharification) molecular weight: HC | | | Preset range |
| | Amino acid composition | | | Preset range |
| | Molar extinction coefficient | | | Preset range |
| | Protein content | | | Preset range |
| secondary and advanced structures | Disulfide bond pairing | | | Preset range |
| | Non-reducing peptide profile | | | Qualitative comparison |
| | Determination of free sulfhydryl (mol/mol) | | | Parameter range |
| | Circular dichroism spectrum | spectrogram | | Qualitative comparison |
| | | CD: α-Helix | | Parameter range |
| | | CD: Beta | | Parameter range |
| | | CD: Turn | | Parameter range |
| | | CD: Random | | Parameter range |
| | DSF(Tm value) | | | Parameter range |
| | Crystal structure | | | Qualitative comparison |
| Post-translational modification | Analysis of glycosylation sites | | | Qualitative comparison |
| | N-sugar modification analysis pattern | | | Qualitative comparison |
| | Proportion of total fucosylated components | | | Parameter range |
| | Ratio of high mannose components | | | Parameter range |
| | Ratio of sialylated components | | | Parameter range |
| | Analysis of oxidation modification ratio | | | Parameter range |
| | Analysis of deamidation modification ratio | | | Parameter range |
| | Analysis of N-terminal pyroglutamic acid cyclization ratio | | | Parameter range |
| | Analysis of the proportion of C-terminal lysine deficiency | | | Parameter range |
| Post-translational modification | cIEF of isoelectric point | Profile of isoelectric point | | Qualitative comparison |
| | | Profile of desaccharification isoelectric point | | Qualitative comparison |
| | | Desaccharification isoelectric point | | Preset range |
| | | After desaccha rification and removal of terminal Lys | Profile | Qualitative comparison |
| | | | Acidic peak | Parameter range |
| | | | Main peak | Parameter range |
| | | | Alkaline peak | Parameter range |
| | IEC-HPLC | Profile of IEC-HPLC | | Qualitative comparison |
| | | IEC-HPLC after desaccharification and removal of terminal Lys | Profile | Qualitative comparison |
| | | | Acidic peak | Parameter range |
| | | | Main peak | Parameter range |
| | | | Alkalin e peak | Parameter range |
| Analysis of polymers and degradation products | SEC-HPLC | Profile | | Qualitative comparison |
| | | Polymer | | Parameter range |
| | | Monomer | | Parameter range |
| | AUC | Profile | | Qualitative comparison |
| | | Monomer% | | Parameter range |
| | DLS | SEC-HPLC-LS spectrum | | Qualitative comparison |
| | | DLS | | Qualitative comparison |
| | CE-SDS | Non reducing CE-SDS spectrum | | Qualitative comparison |
| | | Non reducing CE-SDS : LMW | | Parameter range |
| | | Reducing CE-SDS spectrum | | Qualitative comparison |
| | | Reducing CE-SDS : HC&LC | | Parameter range |
| | | Reducing CE-SDS : NGHC | | Parameter range |
| Thermal stability | High temperature stability at 40°C | | | Qualitative comparison |
| | Acceleration stability at 25°C | | | Qualitative comparison |
| In vitro biological activity | Antigen IL-12 binding activity (specific activity) | | | Parameter range |
| | Antigen IL-12 affinity | | | Parameter range |
| | Antigen IL-23 binding activity (specific activity) | | | Parameter range |
| | Antigen IL-23 affinity | | | Parameter range |
| | Antagonistic activity of IL-12 receptor (specific activity) | | | Parameter range |
| | Antagonistic activity of IL-23 receptor (specific activity) | | | Parameter range |
| | Cell activity (NK-92 MI cells, specific activity) | | | Parameter range |
| | Cell activity (mouse spleen cells, IC50) | | | Parameter range |
| | Fc related receptor affinity | C1q affinity (specific activity) | | Parameter range |
| | | FcyR I | | Parameter range |
| | | FcγR IIa-H131 | | Parameter range |
| | | FcyR IIa-R131 | | Parameter range |
| | | FcyR IIb | | Parameter range |
| | | FcyR IIIa-V158 | | Parameter range |
| | | FcyR IIIa-F158 | | Parameter range |
| | | FcRn pH6.0 | | Parameter range |
| | | FcRn pH7.4 | | Parameter range |
| | Analysis of species specificity | | | Qualitative comparison |
| | Consistency analysis of antigen recognition epitopes | | | Qualitative comparison |

### Example 4: pharmacodynamic studies in mice (preclinical animal level pharmacodynamic evaluation)

In this example, the effect of QX001S on ear epidermal hyperplasia in the mouse ear epidermal hyperplasia model induced by IL-23 was evaluated, and the pharmacodynamic effect on this model was compared between QX001S and the original drug Ustekinumab (the name in this study is QX001S-DZY, a commercially available product in the European Union: batch number FJS1W25). Studies showed that both Ustekinumab and QX001S had significantly inhibited ear skin hyperplasia induced by rhIL-23, which was manifested in decrease in ear thickness and decrease in pathological comprehensive score. The QX001S group and the Ustekinumab group showed a highly similar consistency of pharmacodynamic effect.

Forty-eight C57BL/6 mice were randomly divided into 6 groups, 8 in each group; mice were injected with blank vehicle or rhIL-23 intradermally on the back of the right ear for 8 consecutive days; 1 hour before rhIL-23 injection, they were given QX001S or QX001S-DZY by intraperitoneal injection; ear thickness and ear increasing thickness were measured at day 0, 2, 4, 6 and 8, respectively; on day 8, the right ears of 48 mice were excised to prepare paraffin sections, and stained with HE. The slices were scored from the four aspects of keratinization degree, epidermal thickness, dermal thickness and lymphocyte infiltration (0-4 points for each item), and the scores of these four parts were added together to obtain a comprehensive pathological score (0-16 points).

The experimental results showed that there was no significant difference in the thickness of the right ear, the increasing thickness of the right ear, and the comprehensive pathological score between the QX001S 10mg/kg group and the QX001S-DZY 10mg/kg group (see Table 2 and Table 3 for the specific data).

**Table 2: the thickness and the increasing thickness of the right ear**

| **Administration group** | **Dosage (mg/kg)** | **Thickness of the right ear (mm) day 0** | **Thickness of the right ear (mm) day 8** | **Increasing thickness of the right ear (mm) day 8** |
|---|---|---|---|---|
| - | Control | 0.23±0.01 | 0.39±0.02 | 0.16±0.02 |
| Saline | Model | 0.23±0.01 | 0.69±0.11 ^{##} | 0.46±0.12 ^{##} |
| QX001S | 3mg/kg | 0.23±0.01 | 0.58±0.04 ^{*Δ} | 0.35±0.0 4 ^{*Δ} |
| QX001S | 10mg/kg | 0.23±0.01 | 0.56±0.09 ^{*} | 0.33±0.09 ^{*} |
| QX001S-DZY | 3mg/kg | 0.23±0.01 | 0.67±0.07 | 0.44±0.07 |
| QX001S-DZY | 10mg/kg | 0.23±0.01 | 0.58±0.06^{*} | 0.35±0.07 ^{*} |

| | | | | |
|---|---|---|---|---|
| Data are expressed as mean ± SD. ^{#}p<0.05, ^{##}p<0.01, compared with the control; *p<0.05, **p<0.01, compared with the model; ^{△}p<0.05, ^{△△}p<0.01, QX001S 3mg/kg versus QX001S-DZY 3mg/kg; ^{☆}p<0.05, ^{☆☆}p<0.01, QX001S 10mg/kg versus QX001S-DZY 10mg/kg. | | | | |

**Table 3: pathological score of right ear tissue section after HE staining**

| **Administration group** | **Dosage (mg/kg)** | **Degree of keratinization** | **Thickness of epidermis** | **Thickness of dermis** | **Lymphocytic infiltration** | **Pathological comprehensive score** |
|---|---|---|---|---|---|---|
| - | Control | 0.88±0.64 | 0.25±0.46 | 1.5±0.76 | 0.5±0.53 | 3.13±1.96 |
| Saline | Model | 2.75±0.46 ^{##} | 3.13±0.83 ^{##} | 2.75±0.71 ^{##} | 3±1.07 ^{##} | 11.63±1.06 ^{##} |
| QX001S | 3 | 1.88±0.83 * | 1.75±1.28 ^{*Δ} | 2±0.93 | 2.13±1.36 | 7.75±3.92 * |
| QX001S | 10 | 1.88±0.64 ** | 1.63±1.19 * | 1.5±1.2 * | 1.88±1.36 | 6.88±3.83 ** |
| QX001S-DZY | 3 | 2.5±0.53 | 3.13±0.83 | 2.5±1.07 | 2.63±0.92 | 10.75±2.71 |
| QX001S-DZY | 10 | 1.38±0.52 ** | 1.88±0.99 * | 1.75±1.28 | 1.88±0.83 * | 6.88±2.95** |

| | | | | | | |
|---|---|---|---|---|---|---|
| Data are expressed as mean ± SD. ^{#}p<0.05, ^{##}p<0.01, compared with the control; ^{*}p<0.05, **p<0.01, compared with the model; ^{△}p<0.05, ^{△△}p<0.01, QX001S 3mg/kg versus QX001S-DZY 3mg/kg; *p<0.05, **p<0.01, QX001S 10mg/kg versus QX001S-DZY 10mg/kg. | | | | | | |

### Example 5: In vivo pharmacokinetic studies on Cynomolgus monkeys (preclinical animal level pharmacokinetic evaluation)

In this example, the pharmacokinetic characteristics of QX001S in Cynomolgus monkeys were evaluated in vivo, and the pharmacokinetic parameters of QX001S and the original drug Ulinuzumab (named QX001S-DZY in this study) in Cynomolgus monkeys were compared simultaneously. Studies have shown that both Ustekinumab and QX001S have similar pharmacokinetic parameters and plasma concentration-time variation in Cynomolgus monkeys.

In this example, 36 Cynomolgus monkeys were used, and they were randomly divided into 6 groups, 6 in each group; mice in 3 groups were treated with single subcutaneous injection of QX001S (0.35, 1, 3 mg/kg), mice in one group were treated with single intravenous injection of QX001S (1mg/kg), mice in one group were treated with single subcutaneous injection of QX001S-DZY (1mg/kg), and mice in one group were treated with repeated subcutaneous injections of QX001S (1mg/kg). Serum was collected intravenously from all animals at different time points before and after administration, and the concentration of QX001S and QX001S-DZY in the serum was detected by the validated ELISA method, and the relevant pharmacokinetic parameters were calculated using non-compartmental model.

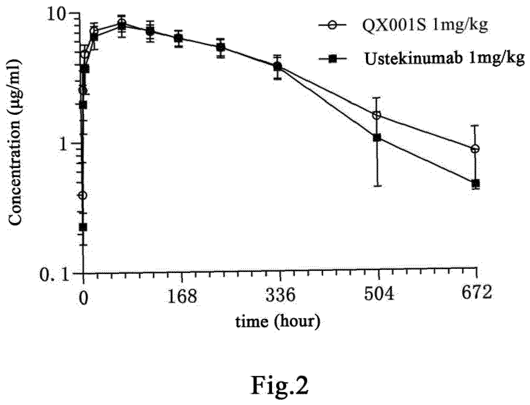

The results of pharmacokinetics showed that there was no significant difference between the main pharmacokinetic parameters of the group of a single subcutaneous injection of 1 mg/kg QX001S and the group of a single subcutaneous injection of 1 mg/kg QX001S-DZY (p>0.05), and the pharmacokinetic parameters and plasma drug concentration time variation of both in Cynomolgus monkeys are similar (shown in Figure 2).

The results of immunogenicity showed that 83% (5/6) of the individuals in the group of a single subcutaneous injection of 1 mg/kg QX001S and the group of a single subcutaneous injection of 1 mg/kg QX001S-DZY had been detected anti-drug antibodies, and the immunogenicity of the two in Cynomolgus monkeys are similar.

### Example 6: pharmacokinetic research in human body (phase I clinical research)

QX001S injection is the world's first biosimilar drug of Ustekinumab injection (trade name Stelara^{®}) entering clinical research. Its phase I clinical study: "a randomized, double-blind, single-dose, parallel comparison of pharmacokinetics between QX001S injection and Ustekinumab injection (EU market: batch number GDS3IME) in healthy Chinese male volunteers" has been successfully completed, and the results of the study have reached all the preset primary and secondary endpoints (National Drug Clinical Trial Registration and Information Disclosure Platform Registration No. CTR20181658). Based on the good results of the phase I clinical study, we are fully preparing to launch the phase III clinical study of QX001S for psoriasis indications. The key test results obtained in the above phase I clinical study include:
1. Pharmacokinetic bioequivalence

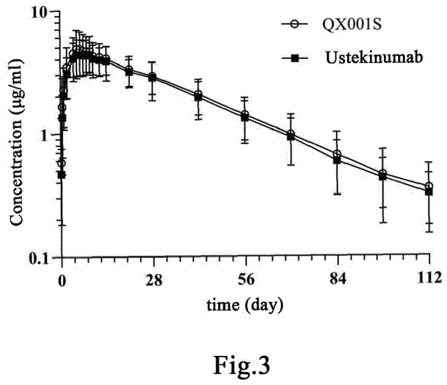

The 90% CIs of the geometric mean ratios of Cmax, AUC0-t and AUC0-inf of QX001S and Ustekinumab were: 100.90%-118.68%, 98.71%-115.26%, 98.49%-115.81%, all falling within the specified equivalence ranges of 80.00%-125.00%, indicating that QX001S injection and Ustekinumab injection have pharmacokinetic bioequivalence (shown in Figure 3).

### 2. Similar security

Among the 177 subjects who entered the safety analysis population in this study, a total of 98 subjects had 228 TEAEs, and none of the subjects had SAEs, nor did they quit due to AEs. The incidence of adverse drug reactions in the QX001S group and the Ustekinumab group were respectively 42.7% and 42.0%, with similar security.

In addition, among the 177 subjects entering the immunogenicity analysis population in this study, there were 11 positive subjects in the QX001S group, with a positive rate of 12.4%, and 21 positive subjects in the Ustekinumab group, with a positive rate of 23.9%.

It should also be noted that the original drug Ustekinumab was recombinantly expressed using SP2/0 cells as host cells, while the biosimilar drug QX001S was recombinantly expressed using CHO-S cells as host cells. Generally speaking, even if the amino acid sequence of the same monoclonal antibody expressed by different host cells is the same, there may be structural differences due to different glycosylation patterns, which may easily cause differences in physical and chemical properties, biological activities, and metabolic behaviors in vivo, etc., resulting in inconsistencies between the biosimilar drugs and the original drug, and cannot pass the consistency evaluation. This is a major difficulty in the development of biosimilar drugs, and it is also an important reason why there is little information about its biosimilars, although the patent protection of Ustekinumab is about to expire. In contrast, the biosimilar drug of Ustekinumab QX001S expressed by the specific CHO-S cell line obtained by screening in the present application not only showed a high degree of consistency with Ustekinumab in preclinical studies, but also passed the evaluation of pharmacokinetic bioequivalence and safety similarity in clinical studies, has become the first biosimilar drug of Ustekinumab to enter the clinical trial and the only one to complete the Phase I clinical trial in China so far. As far as we know, it is also one of the biosimilar drugs of Ustekinumab with the fastest progress in new drug application internationally.

The embodiments of the present application are described above through specific examples, and those skilled in the art can easily understand other advantages and efficiencies of the present application from the contents disclosed in this specification. The present application can also be implemented or applied through other different specific embodiments, and various modifications or changes can be made to the details in this specification based on different viewpoints and applications without departing from the spirit of the present application.

### PCT

### Printed copy (original in electronic form)

| | | |
|---|---|---|
| 0-1 | PCT/RO/134 Form (SAFE) INDICATIONS RELATING TO DEPOSITED MICROORGANISM OR OTHER BIOLOGICAL MATERIAL (PCT Rule 13*bis*) | |
| 0-1-1 | | **CEPCT** |
| | | **Versions 10.25.41(20201201) MT/FOP 20140331/0.20.5.21** |
| | software version | |
| 0-2 | International application No. | |
| 0-3 | Applicant's or agent's file reference | **TFE00358PCT** |
| 1 | The indications made below relate to the deposited microorganism or other biological material referred to in the | |
| 1-1 | | |
| 1-2 | description: Page: | 2 |
| | line: | 8 |
| 1-3 | **IDENTIFICATION OF DEPOSIT** | **China Center for Type Culture Collection** |
| 1-3-1 | | |
| | Name of depositary institution | |
| 1-3-2 | Address of the depositary institution *(including postal code and country)* | **Wuhan University, Wuhan, Hubei Province, China Postcode: 430072** |
| 1-3-3 | | |
| 1-3-4 | Date of deposit | **December 3, 2020 (03.12.2020)** |
| | Accession Number | **CCTCC NO: C2020232** |
| 14 | ADDITIONAL INDICATIONS | Chinese hamster ovary cell CHO-S-4 |
| 1-5 | This note applies to the following designated States | All designated States |
| 1-6 | Instructions submitted separately These instructions will then be submitted to the International Bureau later | |

### To be completed by the receiving Office

| | | |
|---|---|---|
| 0-4 | This form was received together with the international application: (Yes or No) | |
| 0-4-1 | Authorized official | |

### To be filled out by the International Bureau

| | | |
|---|---|---|
| 0-5 | Date of receipt of this form by the International Bureau: | |
| 0-5-1 | Authorized official | |

## Claims

1. A Chinese hamster ovary cell S (CHO-S) cell line, deposited with in the China Center for Type Culture Collection (CCTCC), with a deposit number of CCTCC NO: C2020232;
expressing a fully humanized monoclonal antibody against a p40 subunit shared by human IL-12 and human IL-23;
comprising a heavy chain amino acid sequence of the fully humanized monoclonal antibody is shown as SEQ ID NO: 1, and a light chain amino acid sequence of the fully humanized monoclonal antibody is shown as SEQ ID NO:3;
comprising an expression plasmid that expresses the fully humanized monoclonal antibody against the p40 subunit shared by human IL-12 and human IL-23;
the expression plasmid comprising a nucleotide sequence encoding the heavy chain amino acid sequence of the fully humanized monoclonal antibody shown as SEQ ID NO:2, and a nucleotide sequence encoding the light chain amino acid sequence of the fully humanized monoclonal antibody shown as SEQ ID NO:4.

2. The CHO-S cell line according to claim 1, wherein the expressed fully humanized monoclonal antibody against the p40 subunit shared by human IL-12 and human IL-23 is substantially free of sialylated components.

3. The CHO-S cell line according to claim 1, wherein the expressed fully humanized monoclonal antibody against the p40 subunit shared by human IL-12 and human IL-23 is a biosimilar drug of Ustekinumab.

4. A fully humanized monoclonal antibody against the p40 subunit shared by human IL-12 and human IL-23 expressed by the CHO-S cell line according to any one of claims 1 to 3, wherein the fully humanized monoclonal antibody is a biosimilar drug of Ustekinumab.

5. The fully humanized monoclonal antibody against the p40 subunit shared by human IL-12 and human IL-23 according to claim 5, wherein the fully humanized monoclonal antibody is substantially free of sialylated components.

6. A method for producing a biosimilar drug of Ustekinumab, comprising culturing the CHO-S cell line according to any one of claims 1 to 3, thereby producing the fully humanized monoclonal antibody against the p40 subunit shared by human IL-12 and human IL-23 according to claim 4 or 5.

7. An expression vector, comprising a DNA with a nucleotide sequence shown as SEQ ID NO:2 and a DNA with a nucleotide sequence shown as SEQ ID NO:4.

8. A host cell, comprising the expression vector according to claim 7.
